# EUROPEAN PATENT APPLICATION

(11) **EP 2 859 847 A1**
(43) Date of publication of application: **15.04.2015**
(21) Application number: 14184226.0
(22) Date of filing: 10.09.2014
(51) Int. Cl.: A61B 5/15, A61B 5/151

(54) **Blood collecting apparatus, lancet and lancing device**

(30) Priority: 11.10.2013 TW 102136860
(71) Applicant: YSP Co., Ltd., Hsinchu City 300 (TW)
(72) Inventor: Li, Fu-Yuan, 913 Pingtung County (TW); Hsu, Tien-Tsai, 300 Hsinchu City (TW)
(74) Representative: Casalonga

(57) **Abstract**

The present invention provides a blood collecting apparatus including a lancet and a lancing device. The lancing device is used for inserting the lancet and triggering the lancet to collect blood. The lancing device includes a housing, a lancet holder, a triggering mechanism, a withdrawing mechanism, a distance adjusting mechanism and a cap.

## Description

### BACKGROUND OF THE INVENTION

### 1. Field of the Invention

The present invention relates to a blood collecting apparatus, a lancet, and a lancing device, and more particularly, to a blood collecting apparatus, a lancet, and a lancing device for one time use only.

### 2. Description of the Related Art

A blood collecting apparatus can use its lancet to collect blood sample for further test or processing. For example, the blood collecting apparatus can coordinate with a glucose meter by the obtainment of a blood sample of a user, which in turn tests the blood sample and obtains a glucose value of the user.

Although a blood collecting apparatus using a one-time-use-only lancet has been disclosed in prior art, it requires a user or an operator to manually remove the lancet before a new lancet can be used for installation or testing.

For example, US patent application document No.US20070161960 A1 discloses a sleeve-like lancet device having a needle assembly and a needle carrier, the needle assembly having a pin and a pushing rod corresponding to the pin, the pin having an adjusting bolt connected to one end and a spring connected to another end thereof, wherein the spring abuts against a cap; the needle carrier having a needle disposed therein, and a front and a rear stop disposed on the inner surface thereof, the needle forming a single body with a breakable needle hub, wherein the needle hub comprises a safety bolt; a spring plate set is disposed between the needle and the needle carrier. A user can insert the lancet into the lancing device to prepare the lancing device for use. When the user pushes a button on the lancing device, the pin moves forward to push the lancet to prick and collect blood. As the needle moves forward, the elastic force generated by the spring plate set would retract the needle tip back to the needle hub, moreover, due to the design of the front and rear stop, the needle cannot move to an effective triggering position.

Furthermore, in the application document US20070161960 A1, a specific push button or rod is used for loading the lancet to be triggered, even though the lancet has been used, it still can be loaded and triggered again. Therefore, it is possible that the lancet would be reused if the operator fails to confirm the usability of the lancet, leading to a high risk of cross infection.

Therefore, in view of the demands disclosed in the prior art technique, it is necessary to provide a blood collecting apparatus, a lancing device, and a lancet, which can prevent the lancet from being reused and can simplify the mounting process to satisfy the demands described above.

### SUMMARY OF THE INVENTION

Provided is an easy-to-operate blood collecting apparatus, lancet, and lancing device for a user to directly insert the lancet into lancing device to be triggered and collect blood. After blood is collected, the lancet automatically retracts to the inside of the lancing device, making it impossible to be used again. It is necessary to withdraw the lancet and to insert a new one to let the blood collecting apparatus work again.

Therefore, the present invention provides a blood collecting apparatus, which comprises a lancet and a lancing device. The lancing device is provided for the insertion of the lancet and triggering the lancet to collect blood, the lancing device comprises: a housing, a lancet holder, a triggering mechanism, a withdrawing mechanism, a distance adjusting mechanism, and a cap.

In an embodiment of the present invention, the housing comprises an upper housing and a lower housing. The upper housing and the lower housing are slightly in the shape of a semi ring, respectively. Therefore, the upper housing can combine with the lower housing to form a containing space therebetween.

In an embodiment of the present invention, the lancet holder is provided inside the housing, the lancet holder can move from a to-be-triggered position to a triggered position for triggering the lancet to collect blood, the lancet holder comprises: a lancet fixing mechanism for fixing the lancet when the lancet holder has the lancet inserted therein and moves to the to-be-triggered position; a to-be-triggered position locating device for positioning the lancet holder at the to-be-triggered position; and a retracting mechanism for retracting the lancet holder to a to-be-withdrawn position when the lancet holder had moved to the triggered position and collected blood, the retracting mechanism comprising: an elastic element, wherein the elastic element is pressed to accumulate an elastic force when the lancet holder moves to the trigged position; the elastic element provides the elastic force to retract the lancet holder to the to-be-withdrawn position.

In an embodiment of the present invention, the triggering mechanism comprises: a triggering force providing device for providing a triggering force to move the lancet holder from the to-be-triggered position to the triggered position; a releasing device for releasing the lancet holder from the to-be-triggered position to move the lancet holder to the triggered position with the triggering force.

In an embodiment of the present invention, the withdrawing mechanism comprises: a withdrawing rod for pushing the lancet from a to-be-withdrawn position to a withdrawn position to withdraw the lancet; a restoring device for restoring the withdrawing rod to an initial position after the lancet has been withdrawn; and a force applying device connected with the withdrawing rod, the force applying device being provided for a user to apply a force to move the withdrawing rod.

In an embodiment of the present invention, the distance adjusting mechanism is disposed at a rear end of the lancet holder, the distance adjusting mechanism comprises: an adjusting knob; a turntable sleeved connected with the adjusting knob, wherein the turntable comprises: a protruding portion corresponding to a slanted groove of the housing; and an inner collar corresponding to an outer collar of a top end of the lancet holder; therefore, when the user is turning the adjusting knob, the adjusting knob drives the protruding portion of the turntable to move along the slanted groove so as to adjust a relative distance between the inner collar and the outer collar of the lancet holder, thereby adjusting a moving distance of the lancet holder moving from the to-be-triggered position to the triggered position.

In an embodiment of the present invention, the cap is connected to a front end of the housing, the cap comprises a recess corresponding to the convex rail of the lancet.

In an embodiment of the present invention, the lancet comprises: a needle; a covering portion for covering a rear portion of the needle, the covering portion having a convex rail; and a needle cap for removably covering a front portion of the needle, wherein the needle cap comprises a rotating element for the user to turn the rotating element to remove the needle cap.

### BRIEF DESCRIPTION OF THE DRAWINGS

Fig.1 illustrates a combinational view of a blood collecting apparatus in an embodiment of the present invention;
FIG.2 illustrates an explosive view of the blood collecting apparatus in an embodiment of the present invention;
FIG.3 illustrates a partially combinational view of the blood collecting apparatus in an embodiment of the present invention;
FIG.4 illustrates a view of a lancet holder of the blood collecting apparatus in an embodiment of the present invention;
FIG.5 illustrates a view of a cap of the blood collecting apparatus in an embodiment of the present invention;
FIG.6 illustrates a view of the blood collecting apparatus without the lancet in an embodiment of the present invention;
FIG.7 illustrates a view of the blood collecting apparatus with the lancet inserted in an embodiment of the present invention;
FIG.8 illustrates a view of the blood collecting apparatus with the lancet inserted and the needle cap removed in an embodiment of the present invention;
FIG.9 illustrates a view of the blood collecting apparatus being triggered in an embodiment of the present invention;
FIG.10 illustrates an another view of the blood collecting apparatus being triggered in an embodiment of the present invention;
FIG.11 illustrates a view of the blood collecting apparatus having the lancet retracted in an embodiment of the present invention;
FIG. 12 illustrates a view of withdrawing the lancet of the blood collecting apparatus in an embodiment of the present invention;
FIG.13 illustrates a view of the lancet fixing mechanism in another embodiment of the present invention;
FIG.14 illustrates a view of the lancet holder with the original moving distance;
FIG. 15 illustrates a view of the lancet holder with a decreased moving distance; and
FIG.16 illustrates a view of the lancet holder with an increased moving distance.

### DETAILED DESCRIPTION OF THE PREFERRED EMBODIMENT

The advantages and innovative features of the invention will become more apparent from the following detailed description when taken in conjunction with the accompanying drawings.

The present invention provides a blood collecting apparatus to collect blood and to obtain a blood sample for further test or processing. For example, the blood collecting apparatus can coordinate with a glucose meter by the obtainment of a blood sample of a user, which in turn tests the blood sample and obtains a glucose value of the user. However, the blood collecting apparatus can do more tasks other than working with the glucose meter.

By using the blood collecting apparatus disclosed in the present invention, a user can directly insert the lancet into the to-be-triggered position for subsequent collection of blood. After the lancet is triggered to take blood, the lancet would retract and can be withdrawn. After the lancet is withdrawn, the blood collecting apparatus is restored to the original state for inserting a new lancet to collect blood again.

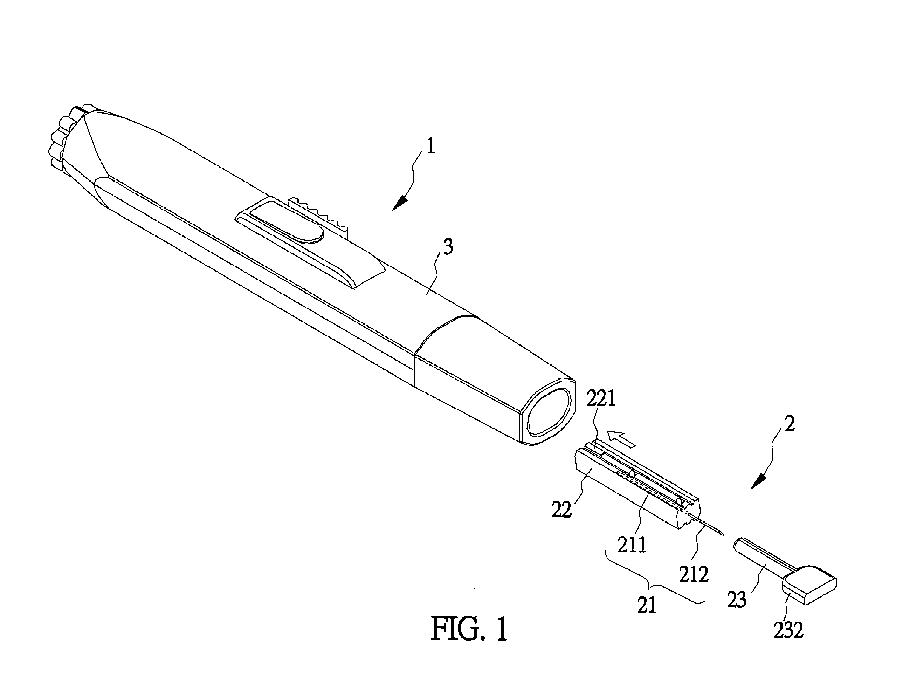

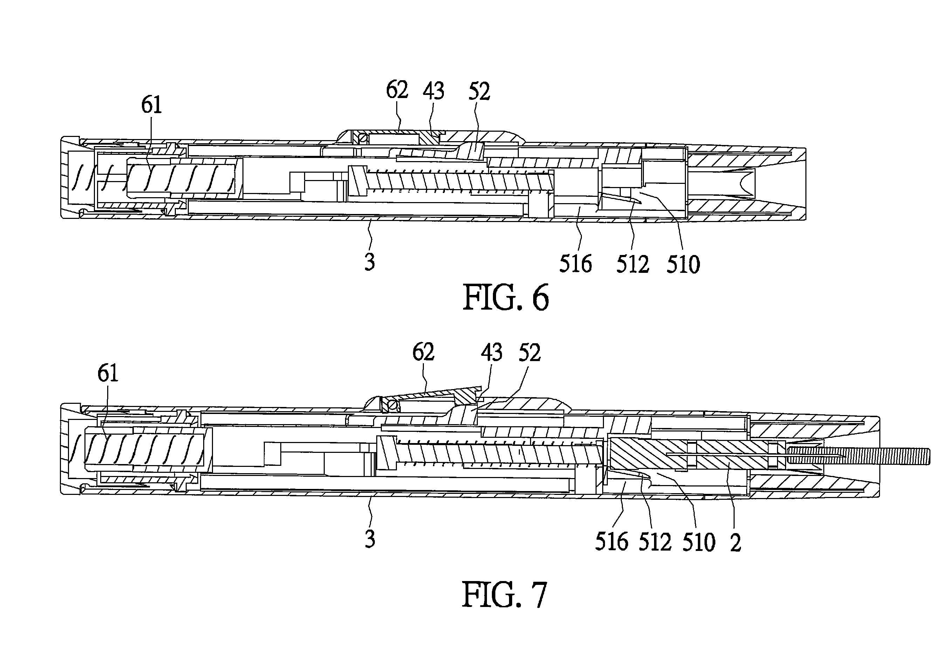

In the following, the structure of the blood collecting apparatus will be described first, and then the operations of blood collecting apparatus will be explained. FIG.1 to FIG.5 illustrate structural views of the blood collecting apparatus in accordance with one embodiment of the present invention, while FIG.6 to FIG. 12 illustrate the operations of the blood collecting apparatus in an embodiment of the present invention.

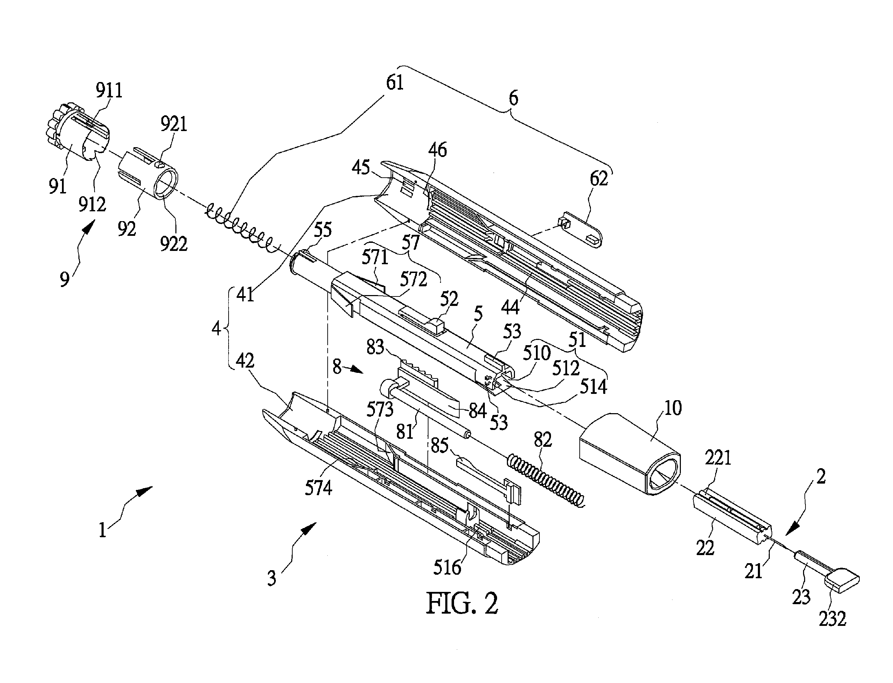

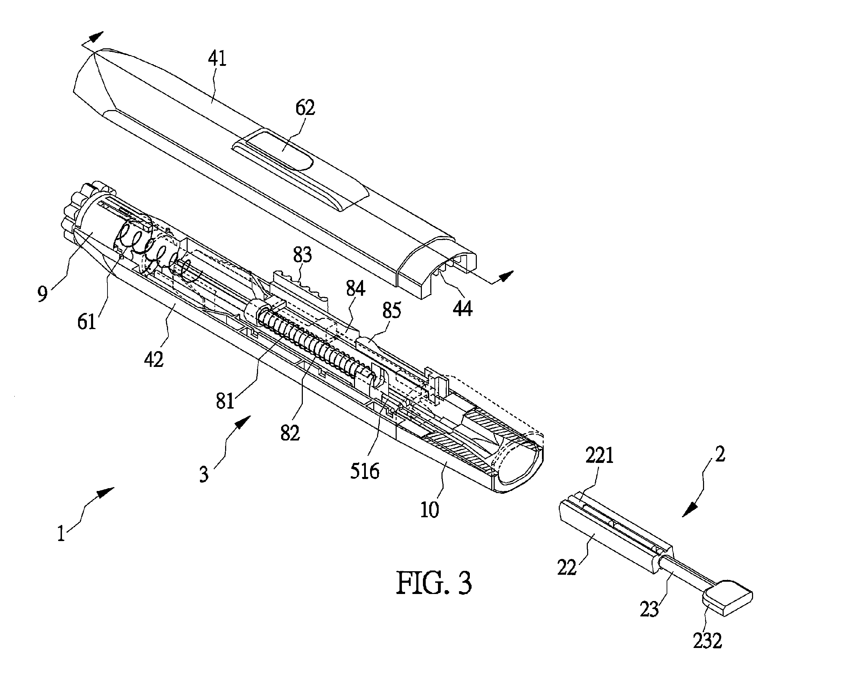

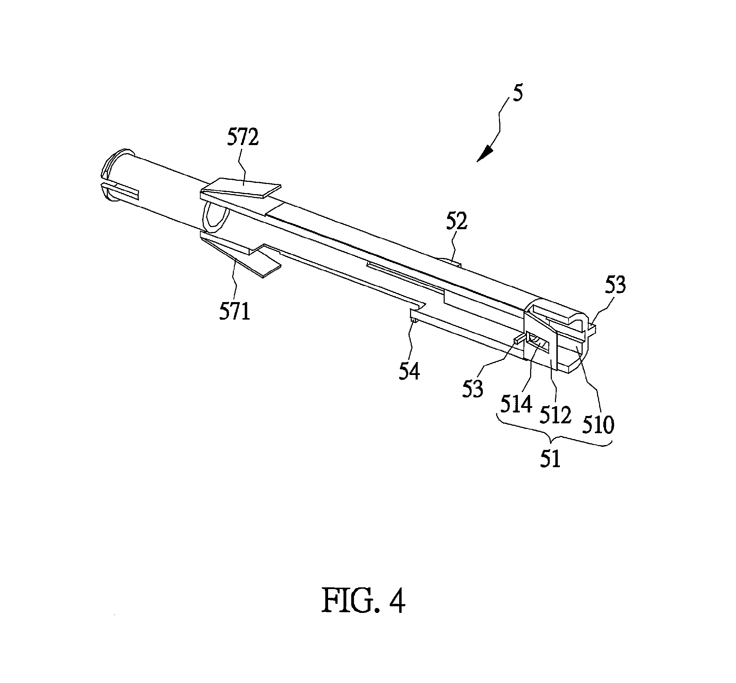

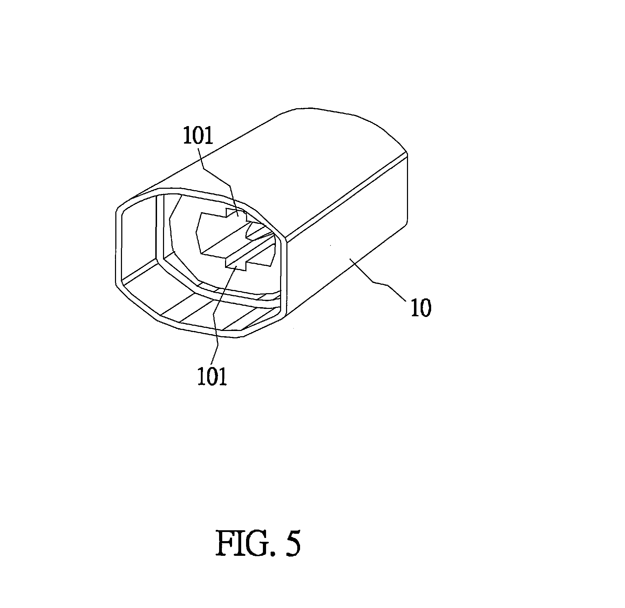

Please refer to FIG.1 to FIG.5, Fig.1 illustrates a combinational view of a blood collecting apparatus in an embodiment of the present invention; FIG.2 illustrates an explosive view of the blood collecting apparatus in an embodiment of the present invention; FIG.3 illustrates a partially combinational view of the blood collecting apparatus in an embodiment of the present invention; FIG.4 illustrates a view of a lancet holder of the blood collecting apparatus in an embodiment of the present invention; and FIG.5 illustrates a view of a cap of the blood collecting apparatus in an embodiment of the present invention.

As shown in FIG.1, in an embodiment of the present invention, the present invention provides a blood collecting apparatus 1, which comprises a lancet 2 and a lancing device 3. The lancing device 3 is provided for inserting the lancet 2 and triggering the lancet 2 to collect blood.

As shown in FIG.1, FIG.2 and FIG.3, the lancet 2 comprises a needle 21, a covering portion 22, and a needle cap 23. The covering portion 22 is configured to cover a rear portion 211 of the needle 21, and the covering portion 22 comprises a convex rail 221 at its top side and bottom side respectively. The needle cap 23 removably covers a front end of the needle 21. The needle cap 23 covers the front portion 212 of the needle 21 when the lancet 2 is not in use; the needle cap 23 is removed to expose the front portion 212 of the needle 21 when the lancet 2 is being used to collect blood. Besides, the needle cap 23 further comprises a rotating element 232 for the user to turn the rotating element 232 to remove the needle cap 23 from outside of the lancing device 3.

As shown in FIG.2 and FIG.3, in an embodiment of the present invention, the lancing device 3 comprises a housing 4, a lancet holder 5, a triggering mechanism 6, a withdrawing mechanism 8, a distance adjusting mechanism 9, and a cap 10.

As shown in FIG.2 and FIG.3, in an embodiment of the present invention, the housing 4 comprises an upper housing 41 and a lower housing 42. The upper housing 41 and the lower housing 42 are slightly in the shape of a semi ring, respectively. Therefore, the upper housing 41 can combine with the lower housing 42 to form a containing space therebetween. In an embodiment of the present invention, the upper housing 41 and the lower housing 42 are made of plastic, but they also can be made of other materials.

As shown in FIG.2 and FIG.3, in an embodiment of the present invention, the lancet holder 5 is disposed in the containing space formed by the upper housing 41 combining with the lower housing 42. The lancet holder 5 is provided for inserting the lancet 2, and the lancet holder 5 can move from a to-be-triggered position to a triggered position to trigger the lancet 2 to collect blood; then the lancet holder 5 can retract to a to-be-withdrawn position after blood is collected. Detailed operations of how the lancet holder 5 moves from the to-be-triggered position to the triggered position and then retracts to the to-be-withdrawn position will be described later.

As shown in FIG.2, FIG.3, and FIG.4, the lancet holder 5 comprises: a lancet fixing mechanism 51, a to-be-triggered position locating device 52, at least one guide rib 53, a retaining bump 54, an outer collar 55, and a retracting mechanism 57. When the lancet holder 5 has the lancet 2 inserted therein and moves to the to-be-triggered position, the lancet fixing mechanism 51 is used for fixing the lancet 2; the to-be-triggered position locating device 52 is configured to position the lancet holder 5 at the to-be-triggered position; the at least one guide rib 53 is used for guiding the lancet holder 5 to move smoothly along with the direction it leads; the retaining bump 54 is used for working with the limit bar 85 of the housing 4 to limit the movement of the lancet holder 5 when the lancet 2 is withdrawn from the lancet holder 5; the outer collar 55 is fixed in the distance adjusting mechanism 9 and works with the distance adjusting mechanism 9 to adjust a moving distance of the lancet holder 5 moving from the to-be-triggered position to the triggered position. Detailed operations of how to adjust the moving distance of the lancet holder 5 will be described later.

In an embodiment of the present invention, the lancet fixing mechanism 51 comprises a clamp opening 510, the clamp opening 510 comprises a clamping piece 512, the clamping piece 512 comprising an opening 514 for the convex rail 221 of the lancet 2 to go through the opening514. The lancet fixing mechanism 51 is disposed to work with the convex step 516 disposed on the lower housing 42. When the lancet 2 is inserted in the lancet holder 5 and is moved to the to-be-triggered position, the convex step 516 presses the clamping piece 512 to move upwards to decrease an aperture of the clamp opening 510 so as to fixedly clamp the lancet 2. When the blood collecting apparatus 1 is at the to-be-triggered state, the lancet fixing mechanism 51 works with the convex step 516 to prevent the user from withdrawing the lancet, while allows the needle cap 23 of the lancet 2 to be moved smoothly, thereby assuring the safety in operation.

In an embodiment of the present invention, the to-be-triggered position locating device 52 can be a bump 52 disposed on the lancet holder 5, when the lancet holder 5 moves to the to-be-triggered position, the bump 52 abuts against the upper housing 41 to position the lancet holder 5 at the to-be-triggered position.

In an embodiment of the present invention, the at least one guide rib 53 corresponds to the at least one recess guide groove 44 of the housing 4 to let the lancet holder 5 move smoothly along the direction guided by the recess guide groove 44.

In an embodiment of the present invention, the outer collar 55 is fixed in the distance adjusting mechanism 9 and blocked by the inner collar 922 of the distance adjusting mechanism 9, so the distance between the outer collar 55 and the inner collar 922 is the moving distance when the lancet holder 5 is being triggered. When the lancet holder 5 is at the to-be-triggered position, the position of the outer collar 55 of the lancet holder 5 is fixed, therefore, the moving distance of the lancet holder 5 moving from the to-be-triggered position to the triggered position can be adjusted by using the distance adjusting mechanism 9 to move the position of the inner collar 922. Detailed operations of how to adjust the moving distance of the lancet holder 5 will be described later.

In an embodiment of the present invention, the retracting mechanism 57 comprises elastic elements 571 and 572 disposed at two sides of the lancet holder 5 respectively. When the lancet holder 5 moves to the triggered position to collect blood; the elastic elements 571 and 572 are deformed by the ramp members 573 and 574 of the lower housing 42 to accumulate an elastic force. When the lancet holder 5 has reached its maximum moving distance, the push force from the spring 61 vanishes, while the elastic elements 571 and 572 release the accumulated elastic force to pull back the lancet holder 5, thereby retracting the lancet holder 5 and the lancet 2 to the to-be-withdrawn position. In an embodiment of the present invention, the lancet 2 retracts back to the inside of the cap 10, or any other designated position.

As shown in FIG.2 and FIG.3, in an embodiment of the present invention, the triggering mechanism 6 is used for triggering the lancet holder 5 to let the lancet holder 5 move from the to-be-triggered position to the triggered position to enable the lancet 2 to collect blood.

In an embodiment of the present invention, the triggering mechanism 6 comprises: a triggering force providing device 61 and a releasing device 62. The triggering force providing device 61 provides a triggering force needed for moving the lancet holder 5 from the to-be-triggered position to the triggered position; the releasing device 62 releases the lancet holder 5 from the to-be-triggered position to let the triggering force move the lancet holder 5 to the triggered position.

In an embodiment of the present invention, the triggering force providing device 61 can be a spring disposed between the distance adjusting mechanism 9 and the lancet holder 5, when the lancet holder 5 moves to the to-be-triggered position, the lancet holder 5 compresses the spring 61 to use a spring force of the spring 61 to provide the triggering force needed for moving the lancet holder 5 to the triggered position. In an embodiment of the present invention, the releasing device 62 can be a button disposed on the upper housing 41, when the button 62 is pressed, the to-be-triggered position locating device 52 can be released to let the lancet holder 5 move by the spring force of the spring 61.

As shown in FIG.2 and FIG.3, in an embodiment of the present invention, the withdrawing mechanism 8 is provided for withdrawing the lancet 2 to replace a new lancet 2 to collect blood again.

In an embodiment of the present invention, the withdrawing mechanism 8 comprises: a withdrawing rod 81, a restoring device 82, and a force applying device 83. The withdrawing rod 81 may be provided for pushing the lancet 2 from a to-be-withdrawn position to a withdrawn position to withdraw the lancet 2. The restoring device 82 is provided for restoring the withdrawing rod 81 to an initial position after the lancet 2 has been withdrawn. The force applying device 83 is connected with the withdrawing rod 81 and provided for a user to apply a force to move the withdrawing rod 81.

In an embodiment of the present invention, the restoring device 82 can be a spring sleeved on the withdrawing rod 81. When the withdrawing rod 81 pushes the lancet 2 to move from the to-be-withdrawn position to the withdrawn position, the spring 82 is compressed to generate an elastic force. Therefore, after the lancet 2 is withdrawn, the elastic force of the spring 82 can bring the withdrawing rod 81 back to the initial position. In an embodiment of the present invention, the withdrawing rod 81 and the restoring device 82 are disposed inside the lancet holder 5, or they can be disposed at any designated positions.

In an embodiment of the present invention, the force applying device 83 is a push button 83, the push button 83 goes through an open slot between the upper housing 41 and the lower housing 42 to protrude out of the housing 4; the push button 83 may be provided for the user to push the push button 83 along the open slot to move the withdrawing rod 81. Furthermore, when the user can't move the push button 83, he/she would find out that the lancing device 3 is in the to-be-triggered state and the lancet 2 can only be withdrawn after being triggered. By pushing the push button 83, this safety design can help the user find out the current state of the lancing device 3.

In an embodiment of the present invention, the withdrawing mechanism further comprises a press bar 84 and a limit bar 85. The press bar 84 moves with the withdrawing rod 81; and the limit bar 85 may be disposed on the lower housing 42. When the withdrawing rod 81 is moving, the press bar 84 moves with the withdrawing rod 81 and presses the limit bar 85, which in turn presses the lancet holder 5 to limit the movement of the lancet holder 5. Furthermore, as shown in FIG.4, the lancet holder 5 comprises a retaining bump 54 working with the limit bar 85. When the press bar 84 works with the withdrawing rod 81 to press the limit bar 85, the limit bar 85 presses the retaining bump 54 to assure that the lancet holder 5 can't be moved, thereby allowing the lancet 2 to be smoothly withdrawn from the lancet holder 5.

As shown in FIG.2 and FIG.3, in an embodiment of the present invention, the distance adjusting mechanism 9 is disposed at a rear end of the lancet holder 5 and is provided for adjusting a moving distance of the lancet holder 5 moving from the to-be-triggered position to the triggered position.

In an embodiment of the present invention, the distance adjusting mechanism 9 comprises an adjusting knob 91 and a turntable 92. The adjusting knob 91 is disposed at a rear end of the housing 4; the turntable 92 is sleeved connected with the adjusting knob 91, wherein the turntable 92 comprises a protruding portion 921 and an inner collar 922. The protruding portion 921 corresponds to a slanted groove 46 of the upper housing 42; and the inner collar 922 corresponds to an outer collar 55 of a top end of the lancet holder 5. When the lancet holder 5 is at the to-be-triggered position, the position of the outer collar 55 is fixed; meanwhile, when the user is turning the adjusting knob 91, the adjusting knob 91 drives the protruding portion 921 of the turntable 92 to move along the slanted groove 46 so as to adjust a relative distance between the inner collar 922 and the outer collar 55 of the lancet holder 5, thereby adjusting a moving distance of the lancet holder 5 moving from the to-be-triggered position to the triggered position. To achieve the aforementioned result, it is noted that, in an embodiment of the present invention, the outer collar (not shown in figure) can be disposed on the turntable 92, and a corresponding inner collar (not shown in figure) can be disposed on top of the lancet holder 5; there are also other possible configurations.

In an embodiment of the present invention, the adjusting knob 91 comprises a positioning bump 911, the upper housing 41 comprises a plurality of positioning grooves 45 working with the positioning bump 911 to position the adjusting knob 91 when the adjusting knob 91 is turned to drive the turntable 92 to move to a specific height. Furthermore, the adjusting knob 91 comprises a concave arc portion 912 working with the protruding portion 921 of the turntable 92.

Since the distance adjusting mechanism 9 can adjust a sliding distance of the lancet holder 5; therefore, the blood collecting apparatus 1 can adjust the moving distance of the lancet 2, thereby allowing the blood collecting apparatus 1 to be applied to people of different ages and requirements.

As shown in FIG.1, FIG.2, FIG.3, and FIG.5, in an embodiment of the present invention, the cap 10 is connected to a front end of the housing 4, the lancet 2 goes through the cap 10 to be inserted in housing 4. The cap 10 comprises a recess 101 corresponding to the convex rail 221 of the lancet 2 to place the lancet 2 firmly in the housing 4. Furthermore, the cap 10 can help to reduce the shaking or swaying when the lancet 2 is triggered to successfully soothe the uncomfortable feeling of the user.

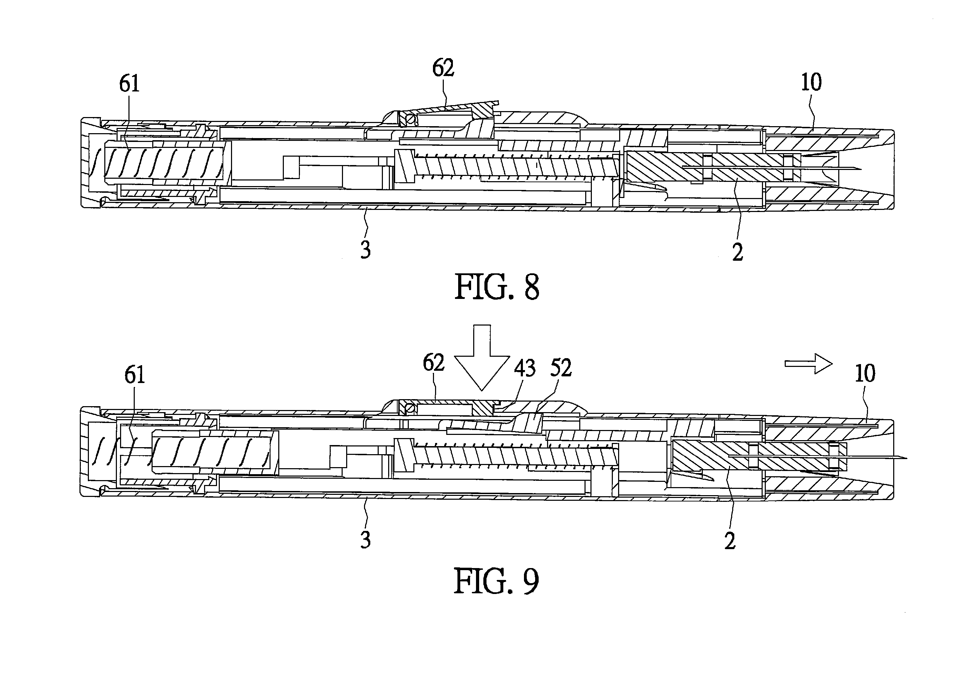

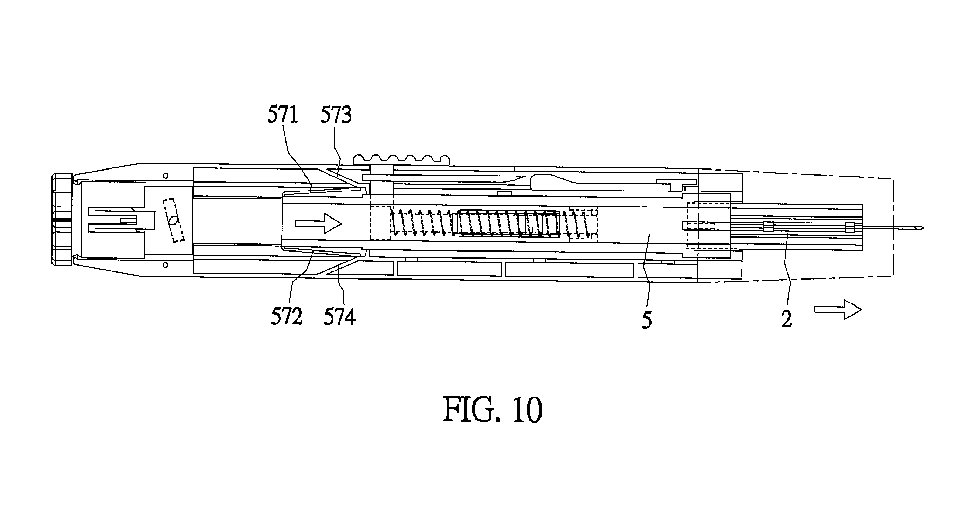

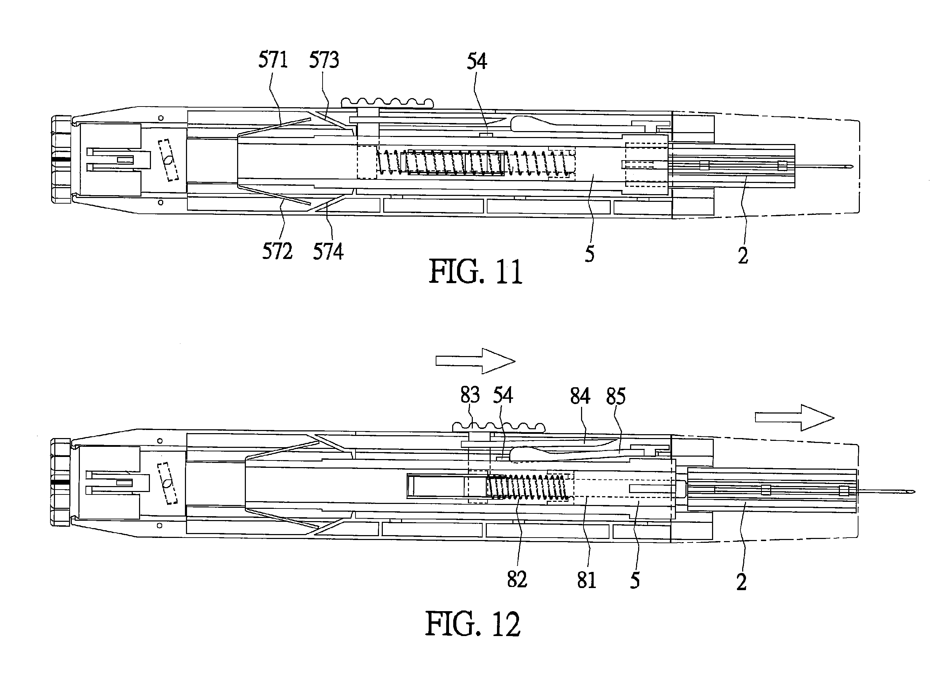

Please refer to FIG.6 to FIG.12 for operations of the blood collecting apparatus in an embodiment of the present invention. FIG.6 illustrates a view of the blood collecting apparatus without the lancet in an embodiment of the present invention; FIG.7 illustrates a view of the blood collecting apparatus with the lancet inserted in an embodiment of the present invention; FIG.8 illustrates a view of the blood collecting apparatus with the lancet inserted and the needle cap removed in an embodiment of the present invention; FIG.9 illustrates a view of the blood collecting apparatus being triggered in an embodiment of the present invention; FIG.10 illustrates an another view of the blood collecting apparatus being triggered in an embodiment of the present invention; FIG.11 illustrates a view of the blood collecting apparatus having the lancet retracted in an embodiment of the present invention; and FIG.12 illustrates a view of withdrawing the lancet of the blood collecting apparatus in an embodiment of the present invention.

As shown in FIG.6, when the lancet 2 is not inserted in the lancing device 3, the clamping piece 512 is not in contact with the convex step 516, and the clamp opening 510 remains its initial aperture; the bump 52 is not in contact with the edge 43 of the upper housing 41; and the spring 61 is no compressed yet.

As shown in FIG.7, when the lancet 2 is inserted in the lancing device 3, the lancet 2 pushes the lancet holder 5 to move upwards, the clamping piece 512 is pressed by the convex step 516 to decrease the aperture of the clamp opening 510 so as to fixedly clamp the lancet 2; the bump 52 moves backward and protrudes out from the edge 43 of the upper housing 41, the bump 52 also pushes the button 62 upwards to protrude out of the housing 4; and the spring 61 is also compressed. At this time, the bump 52 is pushed by the elastic force of the spring 61 to abut against the edge 43 of the upper housing 41, as the bump 52 is stuck with the edge 43 of the upper housing 41, the lancet holder 5 is held at the to-be-triggered position.

Later as shown in FIG.8, when the user desires to collect blood, since the lancet 2 is clamped, the user can smoothly turn the rotating element 232 to remove the needle cap 23 of the lancet 2, at this time the tip of the lancet 2 is still in the cap 10 waiting to be triggered. Meanwhile, the user cannot withdraw the clamped lancet 2, thereby assuring the safety in using the blood collecting apparatus.

As shown in FIG.9, when the user presses the button 62 to trigger the lancet, the bump 52 is recessed from or forced to separate from the edge 43 of the upper housing 41, at this time the spring 61 provides the triggering force needed for moving the lancet holder 5 to the triggered position, thereby allowing the tip of the lancet 2 to protrude out of the cap 10 to collect blood.

As shown in FIG. 10, when the lancet holder 5 has moved to the triggered position to collect blood, the elastic elements 571 and 572 are pressed and deformed by the ramp members 573 and 574 of the lower housing 42 to accumulate an elastic force. When the lancet holder 5 has reached the maximum moving distance, the spring force from the spring 61 vanishes, the elastic elements 571 and 572 will restore their original form and release the accumulated elastic force, as shown in FIG.11, the lancet holder 5 retracts to bring the lancet 2 back to the to-be-withdrawn position. Therefore, the lancet 2 of the blood collecting apparatus 1 can be used for collecting blood, after the lancet 2 has collected blood, the lancet 2 can be retracted and ready for withdrawing.

As shown in FIG.12, after the lancet 2 is triggered and then retracted, the clamp opening 510 restores its original aperture. Then the user can push the push button 83 to move the withdrawing rod 81 forward and to push the lancet 2 out of the lancet fixing mechanism 51 to withdraw the lancet 2. Furthermore, when the withdrawing rod 81 moves, the press bar 84 will move along to abut against the limit bar 85 and limits the limit bar 85 from restoring to the initial position; meanwhile the limit bar 85 presses the retaining bump 54 of the lancet holder 5 to limit the movement of the lancet holder 5. After the lancet 2 is withdrawn, the spring force of the spring 82 restores the withdrawing rod 81 to its initial position. Then the user can insert a new lancet 2 to collect blood again.

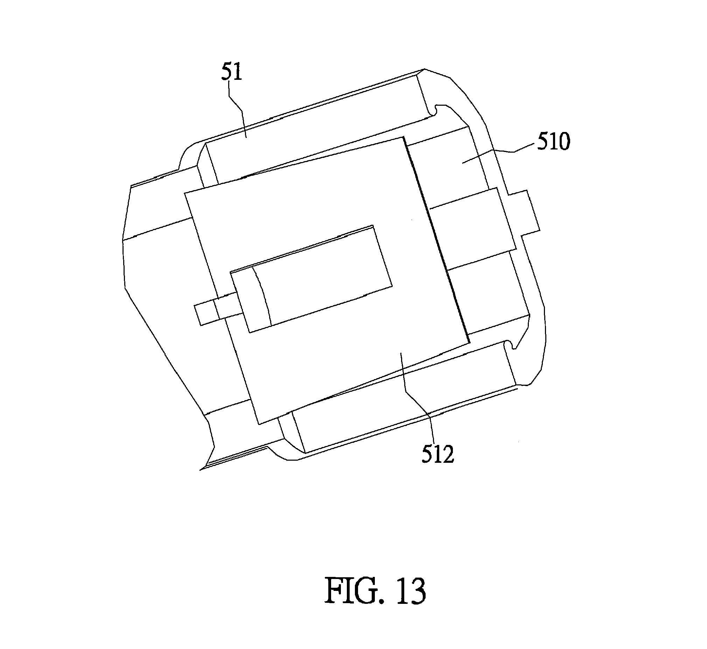

Please now refer to FIG.13 for a view of the lancet fixing mechanism 51 in another embodiment of the present invention. In an embodiment of the present invention, the lancet fixing mechanism 51 can have the whole lower plane of the clamp opening 510 disposed with the clamping piece 512 as shown in FIG.4; or in another embodiment of the present invention, the lancet fixing mechanism 51 can have the lower plane of the clamp opening 510 partially disposed with the clamping piece 512. Therefore, when the lancet 2 is inserted in the lancet fixing mechanism 51, the lancet 2 will be in contact with the lower plane of the clamp opening 510 not disposed with the clamping piece 512. This multi-point contact design will help to further fix the lancet 2 in the lancet fixing mechanism 51. Besides, this design can reduce the risk of the lancet 2 falling off when it is retracting.

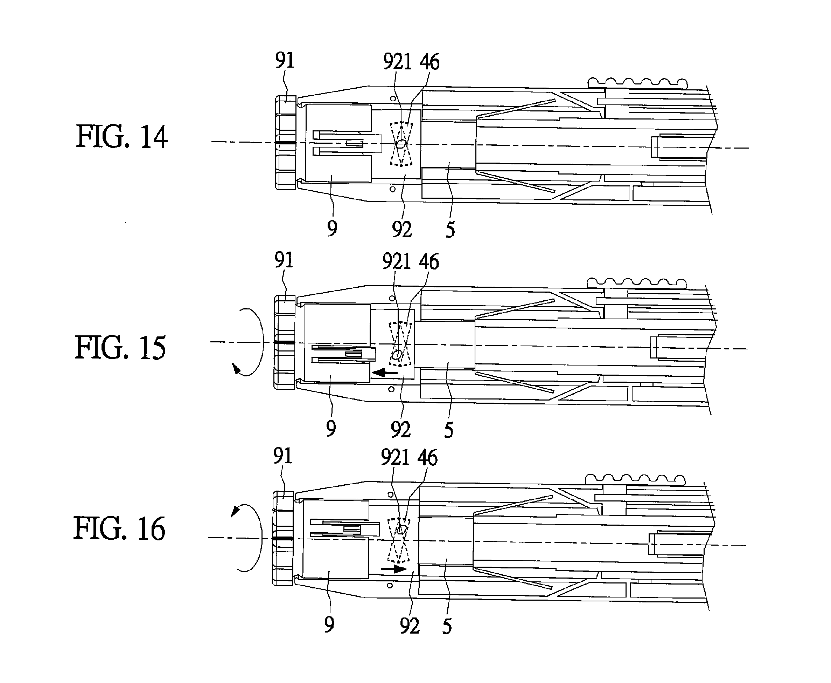

Finally please refer to FIG.14 to FIG.16 for operations of the distance adjusting mechanism 9 of the blood collecting apparatus adjusting the moving distance of the lancet holder 5 in an embodiment of the present invention. FIG. 14 illustrates a view of the lancet holder with the original moving distance; FIG.15 illustrates a view of the lancet holder with a decreased moving distance; and FIG.16 illustrates a view of the lancet holder with an increased moving distance.

As shown in FIG. 14, when the moving distance of the lancet holder 5 is not adjusted, the protruding portion 921 of the turntable 92 is located at the center position of the slanted groove 46. As shown in FIG.15, when the user turns the adjusting knob 91 clockwise, the adjusting knob 91 drives the turntable 92 to move along with the slanted groove 46 to ascend, thereby positioning the protruding portion 921 at an upper position of the slanted groove 46 and also reducing the relative distance between the inner collar 922 and the outer collar 55 of the lancet holder 5. When the lancet holder 5 is triggered, the lancet holder 5 will move until the outer collar 55 of the lancet holder 5 is blocked by the inner collar 922; therefore, the maximum moving distance is the distance between the inner collar 922 and the outer collar 55. Hence, the moving distance of the lancet holder 5 can be shortened by reducing the distance between the inner collar 922 and the outer collar 55. On the contrary, as shown in FIG.16, when the user turns the adjusting knob 91 counterclockwise, the adjusting knob 91 drives the turntable 92 to move along with the slanted groove 46 to descend, thereby positioning the protruding portion 921 at an lower position of the slanted groove 46 and also increasing the relative distance between the inner collar 922 and the outer collar 55 of the lancet holder 5. As described above, the user can adjust the moving distance of the lancet holder 5 to meet different requirements.

As shown in FIG.1, FIG.2 and FIG.3, in an embodiment of the present invention, when assembling the lancing device 3, the upper end of the lancet holder 5 is first inserted to the bottom of the turntable 92, then the outer collar 55 of the lancet holder 5 passes through the inner collar 922 corresponding to the bottom of the turntable 92, thereby allowing the upper end of the lancet holder 5 to be fixed in the turntable 92. Then the withdrawing rod 81 of the withdrawing mechanism 8 is sleeved with one end of the spring 82, and the other end of the spring 82 is fitted in a notch at the lower side of the lower housing 42, and the limit bar 85 is fitted in a notch at a side of the lower housing 42. Afterwards, the sleeved lancet holder 5 is inserted in the lower housing 42 with the withdrawing rod 81 of the withdrawing mechanism 8 disposed in the inner space of the lancet holder 5. The elastic elements 571 and 572 of the retracting mechanism 57 of the lancet holder 5 are right at the positions of the ramp members 573 and 574 of the lower housing 42 respectively. The spring 61 passes through the turntable 92 to be disposed on the upper end of the lancet holder 5. The releasing device 62 is disposed on the upper housing 41, and the adjusting knob 91 is sleeved to the turntable 92. Then the upper housing 41 is combined with the lower housing 42. Finally the user can align the cap 10 to the front end of the housing 4 and press the cap 10 into the housing to combine the upper housing 41, the lower housing 42, and the cap 10 as one unit to complete the assembling process.

When using the blood collecting apparatus 1, the user can insert the lancet 2 through the cap 10 of the lancing device 3 to the lancet holder 5, as the lancet 2 is pushed to the very end of the lancet holder, the user can have the lancing device 3 loaded and the lancet located at the to-be-triggered position. Then the user can turn the needle cap 23 to expose the lancet 2, point the lancing device 3 at the target area (such as finger) and press the button 62, then the lancet 2 would be triggered to collect blood. After the lancet 2 collects blood, it will retract to the inside of the lancing device 3. Since there's no pull lever or pushing rod for the user to reload the lancet 2 to the to-be-triggered position, the user can only withdraw the lancet and replace a new one to be loaded to the to-be-triggered position and to collect blood. Besides, as the total length of the covering portion 22 of the lancet 2 plus the needle cap 23 is longer than the distance between the front end of the cap 10 of the lancing device 3 and the to-be-triggered position of the lancet holder 5 and the total length of the used lancet 2 (without the needle cap 23) is shorter, it is not possible to insert the used lancet again. Therefore, the lancet can be used for one time only to eliminate the possible cross infection risk. Meanwhile, the lancet 2 will automatically retract to the inside of the lancing device 3 to prevent any risk of the lancet 2 mistakenly pricking any one, thereby assuring the safety in the process of blood collection.

It is noted that in one embodiment of the invention, the method for using the lancet to collect the blood sample includes: piercing through the user's skin to collect the blood sample directly; or piercing through the user's skin to leave the blood sample flowing to the surface of the user's skin for being further collected.

It is noted that the above-mentioned embodiments are only for illustration. It is intended that the present invention cover modifications and variations of this invention provided they fall within the scope of the following claims and their equivalents. Therefore, it will be apparent to those skilled in the art that various modifications and variations can be made to the structure of the present invention without departing from the scope or spirit of the invention.

## Claims

1. A lancing device for inserting a lancet and triggering the lancet to collect blood, the lancing device comprising:
a housing;
a lancet holder disposed in the housing, wherein the lancet holder can move from a to-be-triggered position to a triggered position for triggering the lancet to collect blood, the lancet holder comprises:
a lancet fixing mechanism for fixing the lancet when the lancet holder has the lancet inserted therein and moves to the to-be-triggered position;
a to-be-triggered position locating device for positioning the lancet holder at the to-be-triggered position; and
a retracting mechanism for retracting the lancet holder to a to-be-withdrawn position when the lancet holder had moved to the triggered position and collected blood, the retracting mechanism comprising:
an elastic element, wherein the elastic element is pressed by a ramp member of the housing to accumulate an elastic force when the lancet holder moves to the trigged position; the elastic element provides the elastic force to
retract the lancet holder to the to-be-withdrawn position;
a triggering mechanism for triggering the lancet holder to move from the to-be-triggered position to the triggered position to enable the lancet to collect blood,
the triggering mechanism comprising:
a triggering force providing device for providing a triggering force to move the lancet holder from the to-be-triggered position to the triggered position; and
a releasing device for releasing the lancet holder from the to-be-triggered position to let the triggering force move the lancet holder to the triggered
position; and
a withdrawing mechanism for withdrawing the lancet, wherein the withdrawing mechanism comprises:
a withdrawing rod for pushing the lancet from a to-be-withdrawn position to a withdrawn position to withdraw the lancet;
a restoring device for restoring the withdrawing rod to an initial position after the lancet has been withdrawn; and
a force applying device connected with the withdrawing rod, the force applying device being provided for a user to apply a force to move the withdrawing rod.

2. The lancing device as claimed in Claim 1, wherein the lancet comprises a convex rail, the housing has a convex step disposed thereon, and the lancet fixing mechanism comprises:
a clamp opening comprising a clamping piece, the clamping piece comprising an opening for the convex rail to go through the opening; and
wherein the clamping piece is disposed to work with the convex step, when the lancet is inserted in the lancet holder and is moved to the to-be-triggered position, the convex step presses the clamping piece to decrease an aperture of the clamp opening so as to fixedly clamp the lancet.

3. The lancing device as claimed in Claim 1, wherein the to-be-triggered position locating device can be a bump disposed on the lancet holder, when the lancet holder moves to the to-be-triggered position, the bump abuts against the housing to position the lancet holder at the to-be-triggered position.

4. The lancing device as claimed in Claim 1, wherein the triggering force providing device can be a spring, when the lancet holder moves to the to-be-triggered position, the lancet holder compresses the spring to use a spring force of the spring to provide the triggering force needed for moving the lancet holder to the triggered position.

5. The lancing device as claimed in Claim 1, wherein the releasing device can be a button disposed on the housing, when the button is pressed, the to-be-triggered position locating device can be released.

6. The lancing device as claimed in Claim 1, wherein the restoring device of the withdrawing mechanism can be a spring sleeved on the withdrawing rod, the spring provides a spring force for restoring the withdrawing rod to the initial position.

7. The lancing device as claimed in Claim 1, wherein the withdrawing mechanism further comprises:
a press bar moving with the withdrawing rod; and
a limit bar disposed on the housing;
therefore, when the withdrawing rod is moving, the press bar moves with the withdrawing rod and presses the limit bar, which in turn presses the lancet holder to limit the movement of the lancet holder.

8. The lancing device as claimed in Claim 7, wherein the lancet holder further comprises a retaining bump working with the limit bar, when the withdrawing rod is moving, the limit bar presses the retaining bump of the lancet holder to limit the movement of the lancet holder.

9. The lancing device as claimed in Claim 1, wherein the force applying device can be a push button going through an open slot of the housing to protrude out of the housing; the push button is provided for the user to push the push button along the open slot to move the withdrawing rod.

10. The lancing device as claimed in Claim 2, wherein the lancing device comprises a cap connected to a front end of the housing, the cap comprises a recess corresponding to the convex rail of the lancet.

11. The lancing device as claimed in Claim 1, wherein the lancet holder comprises at least one guide rib, the housing comprises at least one guide groove corresponding to the at least one guide rib to let the lancet holder move smoothly along the direction guided by the guide groove.

12. The lancing device as claimed in Claim 1 comprising a distance adjusting mechanism disposed at a rear end of the lancet holder, the distance adjusting mechanism comprising:
an adjusting knob;
a turntable sleeved connected with the adjusting knob, wherein the turntable comprises:
a protruding portion corresponding to a slanted groove of the housing; and
an inner collar corresponding to an outer collar of a top end of the lancet holder;
therefore, when the user is turning the adjusting knob, the adjusting knob drives the protruding portion of the turntable to move along the slanted groove so as to adjust a relative distance between the inner collar and the outer collar of the lancet holder,
thereby adjusting a moving distance of the lancet holder moving from the to-be-triggered position to the triggered position.

13. The lancing device as claimed in Claim 12, wherein the adjusting knob comprises a positioning bump, the housing comprises a plurality of positioning grooves working with the positioning bump to position the adjusting knob when the adjusting knob is turned to move to a specific height.

14. The lancing device as claimed in Claim 12, wherein the adjusting knob comprises a concave arc portion corresponding to the protruding portion of the turntable.

15. A lancet inserted in the lancing device as claimed in Claim 1, to be triggered to collect blood, wherein the lancet comprises:
a needle;
a covering portion for covering a rear portion of the needle, the covering portion having a convex rail corresponding to a recess of the lancing device; and
a needle cap for removably covering a front portion of the needle, wherein the needle cap comprises a rotating element for the user to turn the rotating element to remove the needle cap.
